# EUROPEAN PATENT SPECIFICATION

(11) **EP 2 637 661 B1**
(45) Date of publication and mention of the grant of the patent: **20.12.2017**
(21) Application number: 11782111.6
(22) Date of filing: 07.11.2011
(51) Int. Cl.: A61K 31/4439, A61K 31/517, A61K 39/395, A61K 45/06, A61P 35/00, C07K 16/32, A61K 39/00

(54) **Use of (S)-pyrrolidine-1,2-dicarboxylic acid 2-amide 1-({4-methyl-5-[2-(2,2,2-trifluoro-1,1-dimethyl-ethyl)- pyridin-4-yl]-thiazol-2-yl}-amide) in the treatment of EGFR dependent diseases or diseases that have acquired resistance to agents that target EGFR family members**
Verwendung von (S)-Pyrrolidin-1,2-Dicarbonsäure-2-Amid 1 - ({4-Methyl-5- [2- (2,2,2-Trifluor-1,1-Dimethylethyl)Pyridin-4- yl] Thiazol-2-yl}Amid) zur Behandlung von EGFR-abhängigen Erkrankungen oder Erkrankungen mit erworbener Resistenz gegenüber Wirkstoffen, die auf EGFR-Familienmitglieder gerichtet sind
Utilisation de la 1-({4-méthyl-5-[2-(2,2,2-trifluoro- 1,1-diméthyléthyl)- pyridin-4-yl]thiazole-2-yl}-amide) du 2-amide de l'acide (S)-pyrrolidine-1,2-dicarboxylique dans le traitement de maladies dépendantes d'EGFR ou de maladies qui ont acquis une résistance aux agents qui ciblent des membres de la famille d'EGFR

(30) Priority: 08.11.2010 US 411117 P
(43) Date of publication of application: 18.09.2013
(73) Proprietor: Novartis AG, 4056 Basel (CH)
(72) Inventor: BRACHMANN, Saskia Maria, CH-4002 Basel (CH); FRITSCH, Christine, CH-4002 Basel (CH); MAIRA, Sauveur-Michel, CH-4002 Basel (CH); SCHNELL, Christian René, CH-4002 Basel (CH); GARCIA-ECHEVERRIA, Carlos, F-92210 Saint-Cloud (FR)
(74) Representative: Rudge, Sewkian
(86) International application number: PCT/EP2011/069522
(87) International publication number: WO 2012/062694

(56) References cited:
- WO-A1-2004/096797
- WO-A1-2010/029082
- CARMEL T CHAN ET AL: "Differential sensitivities of trastuzumab (Herceptin(R))-resistant human breast cancer cells to phosphoinositide-3 kinase (PI-3K) and epidermal growth factor receptor (EGFR) kinase inhibitors", BREAST CANCER RESEARCH AND TREATMENT, KLUWER ACADEMIC PUBLISHERS, BO, vol. 91, no. 2, 1 May 2005 (2005-05-01), pages 187-201, XP019274813, ISSN: 1573-7217
- SHE QING-BAI ET AL: "The BAD protein integrates survival signaling by EGFR/MAPK and PI3K/Akt kinase pathways in PTEN-deficient tumor cells.", CANCER CELL OCT 2005 LNKD- PUBMED:16226704, vol. 8, no. 4, October 2005 (2005-10), pages 287-297, XP7920194, ISSN: 1535-6108 cited in the application
- MENDELSOHN JOHN ET AL: "Epidermal growth factor receptor targeting in cancer", SEMINARS IN ONCOLOGY, W.B. SAUNDERS, US, vol. 33, no. 4, 1 August 2006 (2006-08-01) , pages 369-385, XP009096633, ISSN: 0093-7754, DOI: 10.1053/J.SEMINONCOL.2006.04.003

## Description

### Field of the Invention

The present invention relates to a new use of a specific 2-carboxamide cycloamino urea derivative, namely (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide), in the treatment of Epidermal Growth Factor Receptor (EGFR) (including EGFR1 also known as HER1 or Erb-B1; EGFR2 also known as HER2 or Erb-B2; EGFR3 also known as HER3 or Erb-B3; or EGFR4) dependent diseases or diseases that have acquired resistance to agents that target EGFR family members, wherein the disease is a breast cancer, use of said compound for the manufacture of pharmaceutical compositions for the treatment of said disease, combinations of said compound with EGFR modulators, namely EGFR inhibitors, for said use, methods of treating said diseases with said compound, and pharmaceutical preparations for the treatment of said disease comprising said compound, alone or in combination, especially with an EGFR modulator, which is an EGFR inhibitor.

### Background of the Invention

Somatic mutations in the tyrosine kinase domain of EGFR has been associated with the clinical response to EGFR tyrosine kinase inhibitor such as Gefitinib (Iressa®) or Erlotinib (Tarceva®) (Paez et al., 2004, EGFR mutations in lung cancer: correlation with clinical response to gefitinib therapy, Science, vol 304, 1497-1500). The Epidermal Growth Factor Receptor family is composed of four subtypes, including is a member of the ERbB family of receptors including EGFR1 (also known as HER1 or Erb-B1); EGFR2 (also known as HER2 or Erb-B2); and EGFR3 (also known as HER3 or Erb-B3) and EGFR4 which are transmembrane proteins. Acquired resistance to EGFR modulators occurs in patients who initially responded clinically to therapy, but then developed progressive tumors. Refractory response to EGFR kinase inhibitors is exemplified with the secondary resistant mutation T790M (Kobayashi et al.; 2005; EGFR mutation and resistance of non-small cell lung cancer to gefitinib, N. Engl J Med, Vol 352, 786-792), which is comparable to the resistance mutation(s) observed for Gleevec/Glivec or Dasatinib in chronic myelogenous leukemia (CML) (Gorre et al.; 2002; Bcr-Abl point mutants isolated from patients with imatinib mesylate resistant chronic leukemia remain sensitive to inhibitors of the Bcr-Abl chaperone heat shock protein 90, Blood, vol 100, 3041-3044*)* or GIST patients (Antonescu et al.; 2005; Acquired resistance to Imatinib in gastrointestinal stromal tumors occurs through secondary gene mutation, Clin Cancer Res, Vol 11, 4182-4190).

Evidence that activation of the PI3K pathway downstream of activated EGFR exists in the literature. Thus, genetic ablation of the PI3K catalytic subunit (p110) in mouse embryo fibroblast renders cells resistant for transformation by an activated form of EGFR (Zhao et al.; 2006; The p110 alpha isoform of PI3K is essential for proper growth factor signaling and oncogenic transformation, PNAS, vol 103, 16296-16300*).* HER3 (ErbB-3), one of the four member of the EGFR family and partner of HER1 (EGFR1) is often overexpressed in EGFR inhibitors sensitive tumors, and that is correlated with constitutive PI3K recruitment and activation (Engelman et al.; 2005; ErbB-3 mediates phosphoinositide 3-kinase activity in gefitinib-sensitive non small cell lung cancer cell lines, PNAS vol 102, 3788-3793*;* Sergina et al.; 2007; Escape from HER-family tyrosine kinase inhibitor therapy by the kinase-inactive HER 3; Nature; vol 445, 437-41). The genetic and biochemical characterization of tumor biopsies and tumor cell lines harboring EGFR amplification and EGFR inhibitor resistance have revealed a constitutive activation status of the PI3K pathway (Engelman et al.; 2006; Allelic dilution obscures detection of a biologically significant resistance mutation in EGFR amplified lung cancer, The Journal of Clinical Investigation, vol 116, 2695-2706*).*

Chan et al.; 2005 (Differential sensitivities of trastuzumab (Herceptin(R))-resistant human breast cancer cells to phosphoinositide-3 kinase (PI-3K) and epidermal growth factor receptor (EGFR) kinase inhibitors; Breast and Cancer Research and Treatment, vol. 91, 187-201) investigates the mechanism by which Her2-positive breast cancers become resistant to Herceptin, and suggests that combined therapy with Herceptin and one or more inhibitor of PI-3K or EGFR signaling might be a viable therapeutic strategy for Herceptin-resistant human breast carcinomas. She et al.; 2005 (The BAD protein integrates survival signaling by EGFR/MAPK and PI3K/Akt kinase pathways in PTEN-deficient tumor cells; Cancer Cell, vol. 8, 287-297) discloses the inhibition of PI3 Kinase pathway and EGFR as interesting therapeutic strategy in oncology.

Surprisingly, it has been found that a specific 2-carboxamide cycloamino urea derivative, described in WO 2010/029082, provokes strong anti-proliferative activity and an in vivo antitumor response of breast and gastric cancer cell lines with amplified EGFRs and/or mutated EGFR1 as single agent and in combination with EGFR kinase modulators. Therefore, said compound is useful for the treatment of EGFR dependent disease.

### Summary of the Invention

The present invention is defined by the claims.

The present invention relates to the use of a specific compound of formula I (referred to herein as "Compound I", 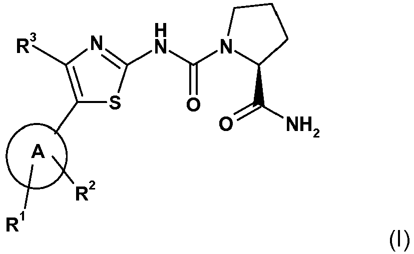 or a salt thereof, wherein
- A: represents a heteroaryl selected from the group consisting of: 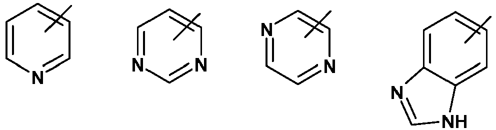
- R¹: represents one of the following substituents: (1) unsubstituted or substituted, preferably substituted C₁-C₇-alkyl, wherein said substituents are independently selected from one or more, preferably one to nine of the following moieties: deuterium, fluoro, or one to two of the following moieties C₃-C₅-cycloalkyl; (2) optionally substituted C₃-C₅-cycloalkyl wherein said substituents are independently selected from one or more, preferably one to four of the following moieties: deuterium, C₁-C₄-alkyl (preferably methyl), fluoro, cyano, aminocarbonyl; (3) optionally substituted phenyl wherein said substituents are independently selected from one or more, preferably one to two of the following moieties: deuterium, halo, cyano, C₁-C₇-alkyl, C₁-C₇-alkylamino, di(C₁-C₇-alkyl)amino, C₁-C₇-alkylaminocarbonyl, di(C₁-C₇-alkyl)aminocarbonyl, C₁-C₇-alkoxy; (4) optionally mono- or di- substituted amine; wherein said substituents are independently selected from the following moieties: deuterium, C₁-C₇-alkyl (which is unsubstituted or substituted by one or more substituents selected from the group of deuterium, fluoro, chloro, hydroxy), phenylsulfonyl (which is unsubstituted or substituted by one or more, preferably one, C₁-C₇-alkyl, C₁-C₇-alkoxy, di(C₁-C₇-alkyl)amino-C₁-C₇-alkoxy); (5) substituted sulfonyl; wherein said substituent is selected from the following moieties: C₁-C₇-alkyl (which is unsubstituted or substituted by one or more substituents selected from the group of deuterium, fluoro), pyrrolidino, (which is unsubstituted or substituted by one or more substituents selected from the group of deuterium, hydroxy, oxo; particularly one oxo); (6) fluoro, chloro;
- R²: represents hydrogen;
- R³: represents (1) hydrogen, (2) fluoro, chloro, (3) optionally substituted methyl, wherein said substituents are independently selected from one or more, preferably one to three of the following moieties: deuterium, fluoro, chloro, dimethylamino;
with the exception of (S)-Pyrrolidine-1,2-dicarboxylic acid 2-amide 1-({5-[2-(tert-butyl)-pyrimidin-4-yl]-4-methyl-thiazol-2-yl}-amide),
and wherein the compound is (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (Compound A),
for the treatment of an EGFR dependent disease, especially malignancies, or EGFR acquired resistance driven disease, and wherein the disease to be treated is breast cancer.

The present invention further relates to the use of the compound A, as defined above, or a salt thereof, for the manufacture of a pharmaceutical preparation for the treatment of an EGFR dependent disease or malignancy or a disease that has acquired resistance to an EGFR modulator, and wherein the disease to be treated is breast cancer.

The present disclosure further relates to the use of a compound of Formula I for the treatment of EGFR dependent diseases or malignancies or a disease that has acquired resistance to an EGFR modulator in combination with other active compounds, for instance, the combination partners as disclosed in WO 2010/029082. Most preferred are EGFR family targeting agents.

The present invention further relates to a combination comprising the compound A and an EGFR modulator selected from the group consisting of gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, TDM1, Zemab®, the Her2 vaccine PX 1041, and the HSP90 inhibitors CNF1010, CNF2024, tanespimycinm, alvespimycin, IPI504, SNX5422 and NVP-AUY922, wherein the active ingredients are present in each case in free form or in the form of a salt, and optionally at least one pharmaceutically acceptable carrier, for simultaneous, separate, or sequential use for the treatment of EGFR dependent disease which is breast cancer.

In another embodiment the present disclosure relates to a method of treating an EGFR dependent disease or a malignancy, preferably a malignancy, that has acquired resistance to EGFR kinase modulators during treatment with said EGFR modulator, comprising administering a therapeutically effective amount of (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (Compound A) or a pharmaceutically acceptable salt thereof, alone or in combination with an EGFR modulator, to a warm-blooded animal in need thereof and wherein the disease to be treated is breast cancer.

In a further embodiment the present invention relates to a pharmaceutical preparation for the treatment of an EGFR dependent disease or a disease that has acquired resistance during treatment with an EGFR modulator comprising (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (Compound A), or a salt thereof and at least one pharmaceutically acceptable carrier, alone or in combination with an EGFR modulator, wherein the disease to be treated is breast cancer.

In a further embodiment, the present invention relates to the use of (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (Compound A), or a salt thereof for the treatment of an EGFR dependent disease or disease that has acquired resistance during treatment with an EGFR modulator, wherein the disease to be treated is breast cancer.

### Description of the Figures

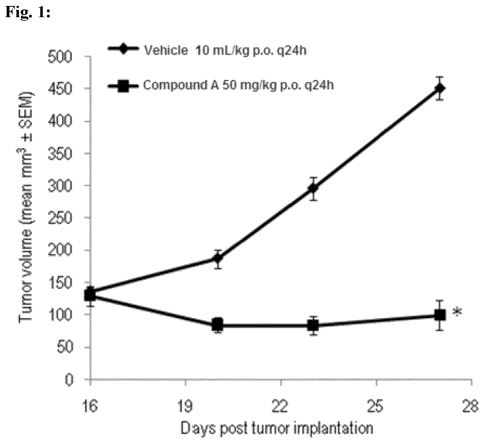

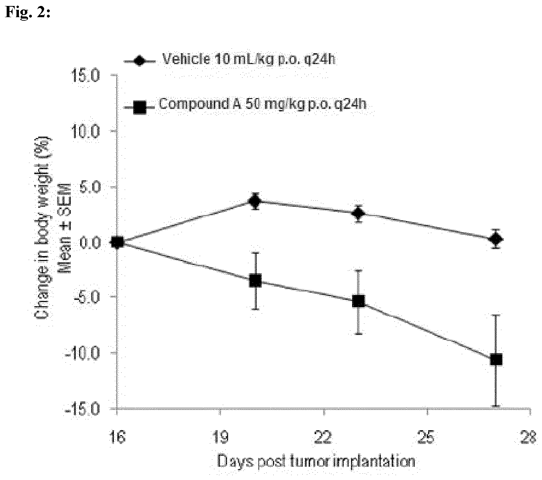

Figure 1 shows the antitumor activity of Compound A against the PIK3CA mutant and ErbB2 amplified breast cancer cell line BT474.
Figure 2 shows the mean body weight of vehicle and Compound A treated groups in mice bearing the orthotopic PIK3CA mutant and ErbB2 amplified breast cancer cell line BT474.
   For the in *vivo* testing in Figures 1 and 2, female athymic mice bearing BT474 orthotopic xenografts were treated with Compound A or vehicle at the indicated doses and schedules. Treatments started 16 days post tumor cells implantation and lasted 11 consecutive days. Statistics on change in tumor volumes were performed with a one-way ANOVA, post hoc Dunnett's (* p<0.05 vs. vehicle controls).

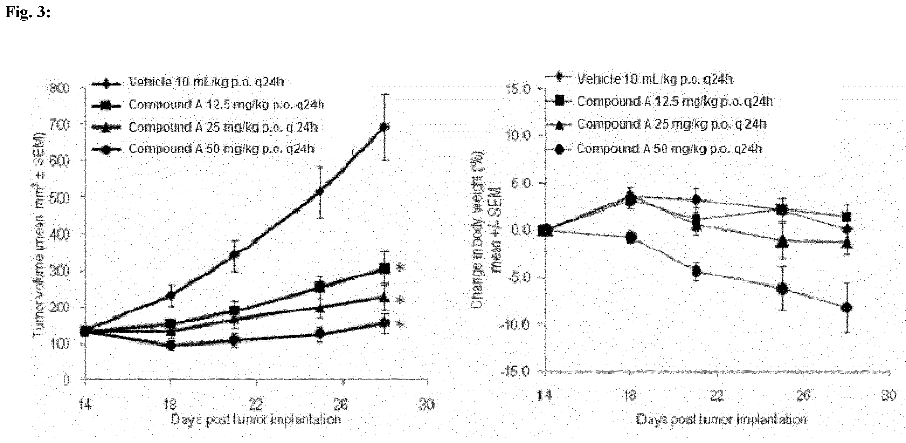

Figure 3 shows the dose-response antitumor activity of 12.5 mg/kg, 25 mg/kg and 50 mg/kg p.o., q24h (i.e., every 24 hours) Compound A against the PIK3CA mutant and ErbB2 amplified breast cancer cell line BT474
   For the in vivo testing of Figure 3, female athymic mice bearing BT474 orthotopic xenografts were treated with Compound A or vehicle at doses and scheduling of 12.5 mg/kg p.o., 25 mg/kg p.o., or 50 mg/kg p.o. Treatments started 14 days post tumor cells implantation and lasted 14 consecutive days. Statistics on change in tumor volumes were performed with a one-way ANOVA, post hoc Dunnett's (* p<0.05 vs. vehicle controls).

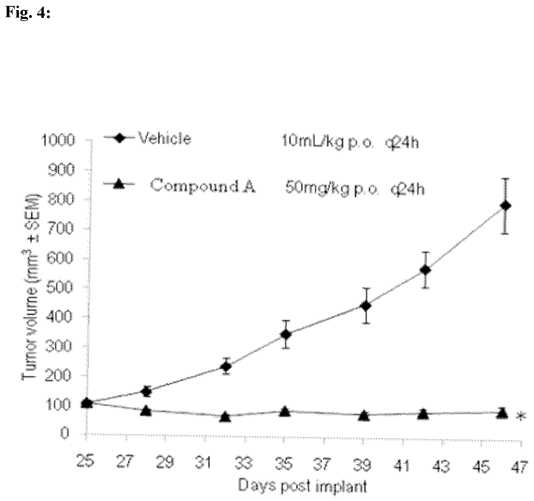

Figure 4 shows the antitumor activity of Compound A against the ErbB2 amplified gastric cancer cell line NCI-N87 (not part of the invention).

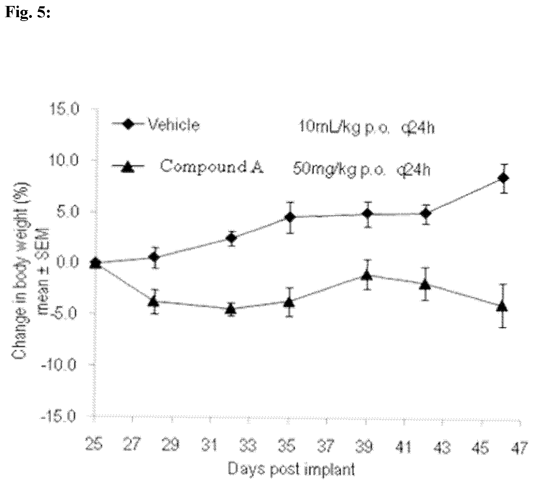

Figure 5 shows the mean body weight of vehicle and Compound A treated groups in mice bearing the subcutaneous ErbB2 amplified gastric cancer cell line NCI-N87 (not part of the invention).
   For the i*n vivo* testing in Figures 3 and 4, Female athymic mice bearing NCI-N87 subcutaneous xenografts were treated with Compound A or vehicle at the indicated doses and schedules. Treatments started 25 days post-tumor cells implantation and lasted 21 consecutive days. Statistics on change in tumor volumes were performed with a one-way ANOVA, post hoc Dunnett's (* p<0.05 vs. vehicle controls).

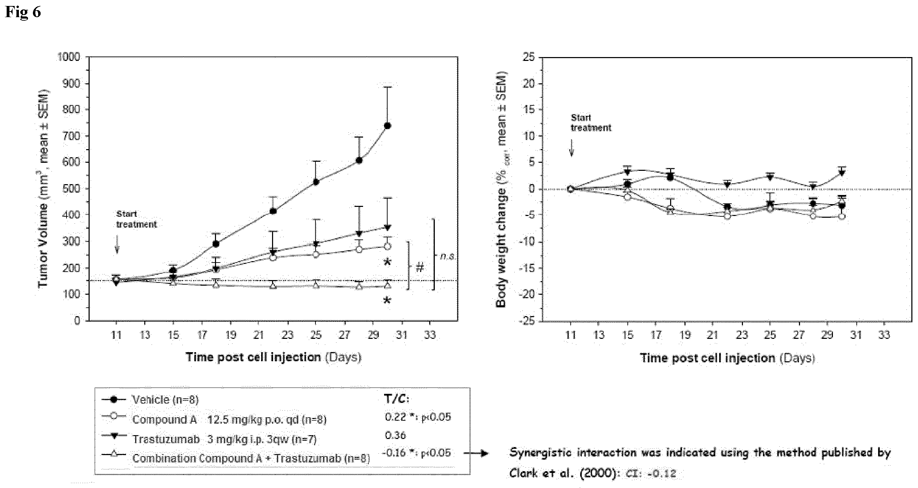

Figure 6 shows the antitumor activity of vehicle, 12.5 mg/kg p.o. qd single agent Compound A, 3 mg/kg i.p. 3 qw of single agent trastuzumab and the combination of Compound A and trastuzumab against the PIK3CA mutant and ErbB2 amplified breast cancer cell line BT474, and the mean corrected changes in body weight (represented by the ratio between body weight at day of measurement and initial body weight at day 11 [both corrected by substraction of primary tumor weight] expressed in percentage for each individual animals) of vehicle, single agent compound A, single agent trastuzumab and the combination of Compound A and trastuzumab treated groups in mice bearing the orthotopic PIK3CA mutant and ErbB2 amplified breast cancer cell line BT474. Values are mean ± SEM; sample size (n = 7-10 mice per group). (*p<0.05, significant inhibition compared to vehicle control group; #: p<0.05, significant inhibition when compared to single agent treatment (Mann-Whitney Rank Sum Test; ns: not significant).\

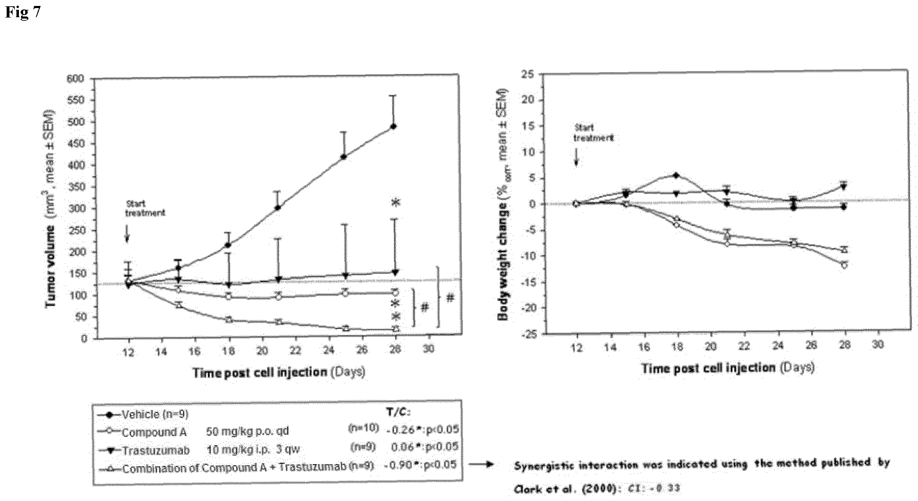

Figure 7 shows the antitumor activity of vehicle, 50 mg/kg p.o. qd of single agent Compound A, 10 mg/kg i.p. 3qd of single agent trastuzumab and the combination of Compound A and trastuzumab against the PIK3CA mutant and ErbB2 amplified breast cancer cell line BT474, and the mean corrected changes in body weight (represented by the ratio between body weight at day of measurement and initial body weight at day 12 [both corrected by substraction of primary tumor weight] expressed in percentage for each individual animals) of vehicle, single agent compound A, single agent trastuzumab and the combination of Compound A and trastuzumab treated groups in mice bearing the orthotopic PIK3CA mutant and ErbB2 amplified breast cancer cell line BT474. Values are mean ± SEM; sample size (n = 9-10 mice per group). (*p<0.05, significant inhibition compared to vehicle control group; #: p<0.05, significant inhibition when compared to single agent treatment (Mann-Whitney Rank Sum Test).

### Detailed Description of the Invention

The following general definitions shall apply in this specification, unless otherwise specified:

"Treatment" includes prophylactic (preventive) and therapeutic treatment as well as the delay of progression of a disease or disorder. The term "delay of progression" as used herein means administration of the combination to patients being in a pre-stage or in an early phase of the proliferative disease to be treated, in which patients for example a preform of the corresponding disease is diagnosed or which patients are in a condition, e.g. during a medical treatment or a condition resulting from an accident, under which it is likely that a corresponding disease will develop.

"Epidermal Growth Factor Receptor dependent diseases" or "EGFR dependent diseases" are especially such disorders or malignancy that respond in a beneficial way (e.g. amelioration of one or more symptoms, delay of the onset of a disease, up to temporary or complete cure from a disease) to the inhibition of a member of the Epidermal Growth Factor Receptor family (where among the diseases to be treated may include proliferative diseases such as cancers or tumor diseases). The Epidermal Growth Factor Receptor family is composed of four members: EGFR1 (also known as HER1 or Erb-B1); EGFR2 (also known as HER2 or Erb-B2); EGFR3 (also known as HER3 or Erb-B3); and EGFR4.

"Pharmaceutical preparation" or "Pharmaceutical composition" refers to a mixture or solution containing at least one therapeutic compound to be administered to a mammal, e.g., a human in order to prevent, treat or control a particular disease or condition affecting the mammal.

"Pharmaceutically acceptable" refers to those compounds, materials, compositions and/or dosage forms, which are, within the scope of sound medical judgment, suitable for contact with the tissues of mammals, especially humans, without excessive toxicity, irritation, allergic response and other problem complications commensurate with a reasonable benefit/risk ratio.

"Salts" (which, what is meant by "or salts thereof" or "or a salt thereof'), can be present alone or in mixture with free compound of the formula (I) and are preferably pharmaceutically acceptable salts. Such salts are formed, for example, as acid addition salts, preferably with organic or inorganic acids, from compounds of formula (I) with a basic nitrogen atom, especially the pharmaceutically acceptable salts. Suitable inorganic acids are, for example, halogen acids, such as hydrochloric acid, sulfuric acid, or phosphoric acid. Suitable organic acids are, e.g., carboxylic acids or sulfonic acids, such as fumaric acid or methansulfonic acid. For isolation or purification purposes it is also possible to use pharmaceutically unacceptable salts, for example picrates or perchlorates. For therapeutic use, only pharmaceutically acceptable salts or free compounds are employed (where applicable in the form of pharmaceutical preparations), and these are therefore preferred. In view of the close relationship between the novel compounds in free form and those in the form of their salts, including those salts that can be used as intermediates, for example in the purification or identification of the novel compounds, any reference to the free compounds hereinbefore and hereinafter is to be understood as referring also to the corresponding salts, as appropriate and expedient. The salts of compounds of formula (I) are preferably pharmaceutically acceptable salts; suitable counter-ions forming pharmaceutically acceptable salts are known in the field.

"Combination" refers to either a fixed combination in one dosage unit form, or a non-fixed combination (or kit of parts) for the combined administration where a compound of the formula (I) and a combination partner (e.g. an other drug as explained below, also referred to as "therapeutic agent" or "co-agent") may be administered independently at the same time or separately within time intervals, especially where these time intervals allow that the combination partners show a cooperative, e.g. synergistic effect. The term "combined administration" or the like as utilized herein are meant to encompass administration of the selected combination partner to a single subject in need thereof (e.g. a patient), and are intended to include treatment regimens in which the agents are not necessarily administered by the same route of administration or at the same time. The term "fixed combination" means that the active ingredients, e.g. a compound of formula (I) and a combination partner, are both administered to a patient simultaneously in the form of a single entity or dosage. The terms "non-fixed combination" or "kit of parts" mean that the active ingredients, e.g. a compound of formula (I) and a combination partner, are both administered to a patient as separate entities either simultaneously, concurrently or sequentially with no specific time limits, wherein such administration provides therapeutically effective levels of the two compounds in the body of the patient. The latter also applies to cocktail therapy, e.g. the administration of three or more active ingredients.

"Therapeutically effective" preferably relates to an amount that is therapeutically or in a broader sense also prophylactically effective against the progression of a proliferative disease.

The present disclosure relates to the use of specific 2-carboxamide cycloamino urea derivatives, alone or in combination, in the treatment of Epidermal Growth Factor Receptor (EGFR) family members (including EGFR1 also known as HER1 or Erb-B1; EGFR2 also known as HER2 or Erb-B2; EGFR3 also known as HER3 or Erb-B3; and EGFR4) dependent diseases.

Specific 2-carboxamide cycloamino urea derivatives which are suitable for the present disclosure, their preparation and suitable pharmaceutical formulations containing the same are described in WO 2010/029082 and include compounds of formula I 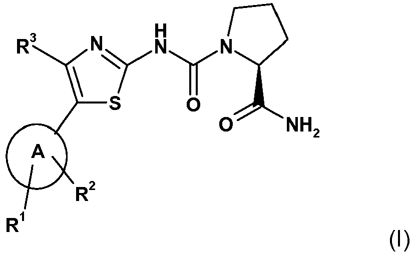 or a salt thereof, wherein
A, R¹ , R², and R³ are as described above, and wherein the compound of Formula (I) is not
((S)-Pyrrolidine-1,2-dicarboxylic acid 2-amide 1-({5-[2-(tert-butyl)-pyrimidin-4-yl]-4-methyl-thiazol-2-yl}-amide).

The radicals and symbols as used in the definition of a compound of formula I have the meanings as disclosed in WO 2010/029082.

A preferred compound of the present invention is (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (COMPOUND A) or a pharmaceutically acceptable salt thereof. The synthesis of (S)-Pyrrolidine-1,2-dicarboxylic acid 2-amide 1-({4-methyl-5-[2-(2,2,2-trifluoro-1,1-dimethylethyl)-pyridin-4-yl]-thiazol-2-yl}-amide) is for instance described in WO 2010/029082 as Example 15.

The ERbB family of receptors including EGFR1 (also known as HER1 or Erb-B1); EGFR2 (also known as HER2 or Erb-B2); and EGFR3 (also known as HER3 or Erb-B3) and EGFR4 are often overexpressed in cellular proliferative disorders or carcinomas. ErbB2 (HER2) is often overexpressed in breast, ovarian and gastric carcinomas. Although effective therapeutic approaches exist against ErbB2, 50% of patients with amplified/ overexpressed HER2 do not respond to ErbB2 modulators such as trastuzumab. The breast cancer BT474 and HBCx-5 cell lines and gastric cancer NCI-N87 cell lines are useful models with ErbB2 amplification and highly tumorigenic *in vivo.* The antitumor activity of PI3K inhibitors such as the compounds of formula I is tested against both ErbB2 driven tumor models.

Surprisingly, administration of Compound A causes *in vivo* tumor grown inhibition in both models. In a panel of breast, CRC and pancreas cell lines containing or not containing ErbB2 amplification, Compound A decreases cell proliferation with a median Gl₅₀ of 139 ± 82 nM in the BT474 breast cancer model and a median Gl₅₀ of 687 ± 132 nM in the HCC1954 breast cancer model and induces cell death in both cell lines that overexpressed ErbB2.

In the orthotopically implanted BT474 breast cancer xenograft model in female athymic nude mice, Compound A produces a statistically significant antitumor effect in the Compound A group treated with doses of 12.5, 25 and 50 mg/kg as compared to the vehicle treated group (p<0.05, ANOVA, post hoc Dunnet's). Compound A administered orally (p.o.) at 50 mg/kg once daily produces a mean tumor change of tumor volume of -29.7 ± 15.6 mm³ (p<0.01, ANOVA and post-hoc Dunnett's) as compared to vehicle (mean tumor change of 315.1 ± 16.4 mm³), with a 23.0% regression in the first experiment. (See Figure 1) Compound A administered orally at 12.5, 25 and 50 mg/kg once daily produces a mean change of tumor volume of 171.8 ± 33.0 mm³ (p<0.01, ANOVA and post-hoc Dunnett's), 95.5 ± 26.5 mm³ (p<0.01, ANOVA and post-hoc Dunnett's), and 20.8 ± 15.9 mm³ (p<0.01, ANOVA and post-hoc Dunnett's) respectively as compared to vehicle (mean change of tumor volume of 557.7 ± 79.0 mm³) in the second experiment. (See Figure 3)

Compound A is well tolerated at 12.5 and 25 mg/kg as demonstrated by the non-statistically significant mean body weight change for the vehicle treated group (0.1 ± 1.2%) and the Compound A treated group (1.4 ± 1.2% and -1.2 ± 1.2% respectively). However, the group treated with 50 mg/kg of Compound A shows a statistically significant mean change of body weight of 10.6 ± 4.1% (p<0.05, using a paired t-test) and 8.2 ± 2.6% (p<0.05, using a paired t-test) in the two experiments. (See Figure 2)

In the subcutaneous NCI-N87 gastric xenograft model in female athymic nude mice, Compound A produces a statistically significant antitumor effect in the Compound A treated group as compared to vehicle (p<0.05, ANOVA), with 11% regression in the experiment. (See Figure 4) The Compound A treated group administered orally (p.o.) at doses of 50 mg/kg once daily produces a mean change of tumor volume of -11.8 ± 17.2 mm³ (p<0.01, ANOVA and post-hoc Dunnett's) as compared to vehicle (mean change of tumor volume of 694.0 ± 93.5 mm³. Compound A at dose of 50 mg/kg. Compound A is well tolerated as demonstrated by the non-statistically significant mean change of body weight for the group treated with 50 mg/kg of Compound A (3.8 ± 2.1%) in the experiment. (See Figure 5)

A compound of formula I, especially Compound A, is therefore useful for the treatment of such EGFR dependent diseases, especially malignancies, or EGFR family members acquired resistance driven diseases. Diseases or malignancies with an established or potential molecular link to dysregulation of EGFR activity are, for instance, described in *"*Mendelsohn and Baselga; Status of Epidermal Growth Factor Receptor Antagonists in the Biology and Treatment of Cancer, Journal of Clinical Oncology, 2787-2799*"; "*Mendelsohn and Baselga; Epidermal Growth Factor Receptor Targeting in Cancer, Seminars in Oncology, Vol 33, 369-385*";* Irmer et al., 2007, EGFR kinase domain mutations - functional impact and relevance for lung cancer therapy, Oncogene, 1-9*;* Roche-Lima et al., EGFR targeting of solid tumors; Cancer Control, 2007, Vol 14 (3), 295-304).

According to the present invention the treatment of the following EGFR dependent disease, especially malignancies, with Compound A, is preferred:
- breast cancer.

Further, the present invention relates to the use of a compound A as described above or a salt thereof for the manufacture of a pharmaceutical preparation for the treatment of an EGFR dependent disease or malignancy, wherein the disease to be treated is breast cancer.

In one embodiment, the present invention relates to the use of (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (Compound A) or a salt thereof for the manufacture of a pharmaceutical preparation for the treatment of a EGFR dependent disease or malignancy or a disease that has acquired resistance to other compounds that target EGFR family members, wherein the disease to be treated is breast cancer.

Compounds that target members of the EGFR family according to the present invention include EGFR family kinase modulators, compounds that alter EGFR expression levels or elicit a cellular immune response linked to the expression of EGFR family members in the tumor cells. As used herein, the term "EGFR modulator" shall mean a compound or drug that is a biological molecule or a small molecule that directly or indirectly modulates EGFR activity or the EGFR signal transduction pathway. Direct or indirect modulation includes activation or inhibition of EGFR activity or the EGFR signal transduction pathway. In one aspect, inhibition refers to inhibition of the binding of EGFR to an EGFR ligand such as, for example, EGF. In another aspect, inhibition refers to inhibition of the kinase activity of EGFR.
The resistance to the treatment with an EGFR modulator can be acquired during treatment with said EGFR modulator or can be due to a mutation or mutations in the protein.

Preferable EGFR modulators exhibit their activity as inhibitors of EGFR functional activity. Compounds that target members of the EGFR family according to the present invention include without limitation gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, TDM1, Zemab®, the Her2 vaccine PX 1041, and the HSP90 inhibitors CNF1010, CNF2024, tanespimycinm alvespimycin, IPI504, SNX5422 and NVP-AUY922.

EGFR modulators include, for example, EGFR-specific ligands, small molecule EGFR inhibitors, and EGFR monoclonal antibodies. In one aspect, the EGFR modulator inhibits EGFR activity and/or inhibits the EGFR signal transduction pathway. In another aspect, the EGFR modulator is an EGFR monoclonal antibody that inhibits EGFR activity and/or inhibits the EGFR signal transduction pathway.

EGFR modulators include biological molecules or small molecules. Biological molecules include all lipids and polymers of monosaccharides, amino acids, and nucleotides having a molecular weight greater than 450. Thus, biological molecules include, for example, oligosaccharides and polysaccharides; oligopeptides, polypeptides, peptides, and proteins; and oligonucleotides and polynucleotides. Oligonucleotides and polynucleotides include, for example, DNA and RNA.

Biological molecules further include derivatives of any of the molecules described above. For example, derivatives of biological molecules include lipid and glycosylation derivatives of oligopeptides, polypeptides, peptides, and proteins.

Derivatives of biological molecules further include lipid derivatives of oligosaccharides and polysaccharides, e.g., lipopolysaccharides. Most typically, biological molecules are antibodies, or functional equivalents of antibodies. Functional equivalents of antibodies have binding characteristics comparable to those of antibodies, and inhibit the growth of cells that express EGFR. Such functional equivalents include, for example, chimerized, humanized, and single chain antibodies as well as fragments thereof.

Functional equivalents of antibodies also include polypeptides with amino acid sequences substantially the same as the amino acid sequence of the variable or hypervariable regions of the antibodies. An amino acid sequence that is substantially the same as another sequence, but that differs from the other sequence by means of one or more substitutions, additions, and/or deletions, is considered to be an equivalent sequence. Preferably, less than 50%, more preferably less than 25%, and still more preferably less than 10%, of the number of amino acid residues in a sequence are substituted for, added to, or deleted from the protein.

The functional equivalent of an antibody is preferably a chimerized or humanized antibody. A chimerized antibody comprises the variable region of a non-human antibody and the constant region of a human antibody. A humanized antibody comprises the hypervariable region (CDRs) of a non-human antibody. The variable region other than the hypervariable region, e.g., the framework variable region, and the constant region of a humanized antibody are those of a human antibody.

Suitable variable and hypervariable regions of non-human antibodies may be derived from antibodies produced by any non-human mammal in which monoclonal antibodies are made. Suitable examples of mammals other than humans include, for example, rabbits, rats, mice, horses, goats, or primates.

Functional equivalents further include fragments of antibodies that have binding characteristics that are the same as, or are comparable to, those of the whole antibody. Suitable fragments of the antibody include any fragment that comprises a sufficient portion of the hypervariable (i.e., complementarity determining) region to bind specifically, and with sufficient affinity, to EGFR tyrosine kinase to inhibit growth of cells that express such receptors.

Such fragments may, for example, contain one or both Fab fragments or the F(ab').sub.2 fragment. Preferably, the antibody fragments contain all six complementarity determining regions of the whole antibody, although functional fragments containing fewer than all of such regions, such as three, four, or five CDRs, are also included.

In one aspect, the fragments are single chain antibodies, or Fv fragments. Single chain antibodies are polypeptides that comprise at least the variable region of the heavy chain of the antibody linked to the variable region of the light chain, with or without an interconnecting linker. Thus, Fv fragment comprises the entire antibody combining site. These chains may be produced in bacteria or in eukaryotic cells.

The antibodies and functional equivalents may be members of any class of immunoglobulins, such as IgG, IgM, IgA, IgD, or IgE, and the subclasses thereof.

In addition to the biological molecules discussed above, the EGFR modulators useful in the invention may also be small molecules. Any molecule that is not a biological molecule is considered herein to be a small molecule. Some examples of small molecules include organic compounds, organometallic compounds, salts of organic and organometallic compounds, saccharides, amino acids, and nucleotides. Small molecules further include molecules that would otherwise be considered biological molecules, except their molecular weight is not greater than 450 Da. Thus, small molecules may be lipids, oligosaccharides, oligopeptides, and oligonucleotides and their derivatives, having a molecular weight of 450 Da or less.

In particular, the present invention relates to the treatment of a disease or malignancy that is dependent on EGFR family members or has acquired resistance during treatment with an EGFR modulator, with Compound A or a salt thereof. Possible EGFR modulators that upon treatment can result in resistance are, for instance, gefitinib, erlotinib, lapatinib, cetuximab, nimotuzumab, panitumumab, trastuzumab; and TDM1.

In a further embodiment, the present invention relates to the use of compound A for the treatment of EGFR dependent disease or malignancy or a disease that has acquired resistance to an EGFR modulator ("EGFR acquired resistance diseases") in combination with other active compounds, for instance, the combination partners as disclosed in WO 2010/029082. More preferred combination partners for this aspect of the invention are EGFR family targeting agents such as, and without limitation to gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, TDM1, Zemab®, the Her2 vaccine PX 1041, and the HSP90 inhibitors CNF1010, CNF2024, tanespimycinm alvespimycin, IPI504, SNX5422 and NVP-AUY922.

In a further embodiment, the present invention also relates to a combination comprising a (S)-Pyrrolidine-1,2-dicarboxylic acid 2-amide 1-({4-methyl-5-[2-(2,2,2-trifluoro-1,1-dimethylethyl)-pyridin-4-yl]-thiazol-2-yl}-amide) (Compound A), and an EGFR modulator selected from the group consisting of gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, TDM1, Zemab®, the Her2 vaccine PX 1041, and the HSP90 inhibitors CNF1010, CNF2024, tanespimycinm alvespimycin, IPI504, SNX5422 and NVP-AUY922, wherein the active ingredients are present in each case in free form or in the form of a salt, and optionally at least one pharmaceutically acceptable carrier, for simultaneous, separate or sequential use for treatment of an EGFR dependent disease, wherein the disease is breast cancer.

In particular, the present invention relates to a combination of 2(S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) and an EGFR modulator selected from the group consisting of gefitinib, erlotinib, lapatinib, cetuximab, nimotuzumab, panitumumab, trastuzumab and TDM1, wherein the active ingredients are present in each case in free form or in the form of a pharmaceutically acceptable salt, and optionally at least one pharmaceutically acceptable carrier; for simultaneous, separate or sequential use for the treatment of breast cancer.

In another embodiment the present invention relates to a method of treating an EGFR dependent disease or a malignancy, preferably a malignancy, that has acquired resistance to EGFR kinase modulators during treatment with said EGFR modulator, comprising administering a therapeutically effective amount of (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (Compound A) or a pharmaceutically acceptable salt thereof, alone or in combination with an EGFR modulator, to a warm-blooded animal in need thereof and wherein the disease to be treated is breast cancer.

The disease to be treated by this method is breast cancer.

In a further embodiment the present invention relates to a pharmaceutical preparation for the treatment of an EGFR dependent disease or a disease that has acquired resistance during treatment with an EGFR modulator comprising (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (Compound A), or a salt thereof and at least one pharmaceutically acceptable carrier, alone or in combination with an EGFR modulator.

The disease to be treated by this pharmaceutical preparation is breast cancer.

In a further embodiment, the present invention relates to the use of (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) (Compound A), or a salt thereof for the treatment of an EGFR dependent disease or disease that has acquired resistance during treatment with an EGFR modulator, wherein the disease to be treated is breast cancer.

The disease to be treated by this compound, alone or in combination with an EGFR modulator, is preferentially breast cancer.

A compound of the formula (I) may also be used to advantage in combination with known therapeutic processes, for example, the administration of hormones or, especially, radiation. A compound of formula (I) may in particular be used as a radiosensitizer, especially for the treatment of tumors which exhibit poor sensitivity to radiotherapy.

Treatment in accordance with the invention may be symptomatic or prophylactic.

A compound of formula I can be administered alone or in combination with one or more other therapeutic compounds, possible combination therapy taking the form of fixed combinations or the administration of a compound of the invention and one or more other therapeutic compounds being staggered or given independently of one another, or the combined administration of fixed combinations and one or more other therapeutic compounds.

The dosage of the active ingredient depends upon a variety of factors including type, species, age, weight, sex and medical condition of the patient; the severity of the condition to be treated; the route of administration; the renal and hepatic function of the patient; and the particular compound employed. A physician, clinician or veterinarian of ordinary skill can readily determine and prescribe the effective amount of the drug required to prevent, counter or arrest the progress of the condition. Optimal precision in achieving concentration of drug within the range that yields efficacy requires a regimen based on the kinetics of the drug's availability to target sites. This involves a consideration of the distribution, equilibrium, and elimination of a drug.

The compounds of the invention may be administered by any conventional route, in particular parenterally, for example in the form of injectable solutions or suspensions, enterally, e.g. orally, for example in the form of tablets or capsules, topically, e.g. in the form of lotions, gels, ointments or creams, or in a nasal or a suppository form. Topical administration is e.g. to the skin. A further form of topical administration is to the eye.

Pharmaceutical compositions comprising a compound of the invention in association with at least one pharmaceutical acceptable carrier or diluent may be manufactured in conventional manner by mixing with a pharmaceutically acceptable carrier or diluent.

The pharmaceutical compositions are comprising an amount effective in the treatment of one of the above-mentioned disorders, of compound A or a salt thereof together with pharmaceutically acceptable carriers that are suitable for topical, enteral, for example oral or rectal, or parenteral administration and that may be inorganic or organic, solid or liquid. There are pharmaceutical compositions used for oral administration especially tablets or gelatin capsules that comprise the active ingredient together with diluents, for example lactose, dextrose, mannitol, and/or glycerol, and/or lubricants and/or polyethylene glycol. Tablets may also comprise binders, for example magnesium aluminum silicate, starches, such as corn, wheat or rice starch, gelatin, methylcellulose, sodium carboxymethylcellulose and/or polyvinylpyrrolidone, and, if desired, disintegrators, for example starches, agar, alginic acid or a salt thereof, such as sodium alginate, and/or effervescent mixtures, or adsorbents, dyes, flavorings and sweeteners. It is also possible to use the pharmacologically active compounds of the present invention in the form of parenterally administrable compositions or in the form of infusion solutions. The pharmaceutical compositions may be sterilized and/or may comprise excipients, for example preservatives, stabilizers, wetting compounds and/or emulsifiers, solubilisers, salts for regulating the osmotic pressure and/or buffers. The present pharmaceutical compositions, which may, if desired, comprise other pharmacologically active substances are prepared in a manner known per se, for example by means of conventional mixing, granulating, confectioning, dissolving or lyophilizing processes, and comprise approximately from 1% to 99%, especially from approximately 1% to approximately 20%, active ingredient(s).

The following Examples illustrate the invention described above; they are not, however, intended to limit the scope of the invention in any way. The beneficial effects of the pharmaceutical combination of the present invention can also be determined by other test models known as such to the person skilled in the pertinent art.

### Example 1 - Effect of Compound A in BT474 breast cancer xenograft model

Experiments were performed in female HsdNpa:Athymic Nude-nu mice approximately 8-12 weeks of age at treatment start. All animals were housed under Optimized Hygienic conditions in Makrolon type III cages (max. 10 animals per cage) with free access to food and water.

BT-474 cells, which are human breast ductal carcinoma cells and ErbB2 amplified with a PIK3CA mutation K111N, were grown in DMEM culture medium containing 4.5g/l glucose supplemented with 10% heat-inactivated FCS, 2mM L-glutamine, 1 mM sodium pyruvate and incubated at 37°C in a 5% CO₂ humidified atmosphere. Cell culture reagents were purchased from BioConcept (Allschwil, Switzerland).

BT-474 tumors were established in vivo by injection 3x10⁶ cells in 30µl HBSS (Sigma #H8264) containing 1mg/ml Matrigel (BD # 34234) orthotopically into the 3^{rd} mammary gland on the right side of the animals. The efficacy experiments started when the tumors reached an average size of approximately 130 mm³ (day 14-16 post cell injection). The PK/PD experiment was performed when the tumors reached a size of 150-250m m³ (day 22 after cell injection). On the day of cell injection, a 0.025 17β Estradiol pellent has been implanted subcutaneously on the left flank into each experimental animal under Forene(R) inhalation narcosis.

Compound A was formulated in NMP/PEG300/Solutol HS15/water (10:30:20:40% vol/vol). The Compound was fully dissolved in NMP. Then, PEG300 and liquified Solutol were added (in sequence with vortexing after addition of each reagent). Water was added immediately prior to administration to the animals. Compound A or vehicle was administered orally at a volume of 10ml/kg.

Tumor volumes were measured with calipers and determined according to the formula: length x diameter² x π/6. Antitumor activity is expressed as T/C % (mean change of tumor volume of treated animals / mean change of tumor volume of control animals) x 100. Regressions (%) were calculated according to the formula ((mean tumor volume at end of treatment-mean tumor volume at start of treatment)/mean tumor volume at start of treatment) x 100. Body weights and tumor volumes were recorded twice a week.

Where applicable, data is presented as mean ± SEM. For all tests, the level of significance was set at p<0.05. For tumor volumes, comparisons between treatment groups and vehicle control group were done using one-way ANOVA followed by Dunnett's test. The level of significance of body weight change within a group between the start and the end of the treatment period was determined using a paired t-test. Comparisons of delta body weights between treatment and vehicle control groups were performed by a one-way ANOVA followed by a post hoc Dunnett's test.

In the first experiment, Compound A was orally administered daily to BT-474 orthotopic xenografts, tumor-bearing nude mice at a dose of 50mg/kg. Vehicle controls consisted of animals receiving daily an oral administration of 10 ml/kg of a mixture of NMP/ PEG300/Solutol HS15/ water (10:30:20:40 % vol/vol). Compound A administered orally at 50 mg/kg once daily produces a mean tumor change of tumor volume of -29.7 ± 15.6 mm³ (p<0.01, ANOVA and post-hoc Dunnett's) as compared to vehicle (mean tumor change of 315.1 ± 16.4 mm³), with a 23.0% regression in the first experiment.

In a second experiment, Compound A was orally administered daily to BT-474 orthotopic xenografts, tumor-bearing nude mice at doses of 12.5, 25 and 50 mg/kg. Vehicle controls consisted of animals receiving daily an oral administration of 10 ml/kg of a mixture of NMP/ PEG300/Solutol HS15/ water (10:30:20:40 % vol/vol). Compound A produces a statistically significant antitumor effect in the Compound A group treated with doses of 12.5, 25 and 50 mg/kg as compared to the vehicle treated group (p<0.05, ANOVA, post hoc Dunnet's). (See Figure 1). Compound A administered orally at 12.5, 25 and 50 mg/kg once daily produces a mean change of tumor volume of 171.8 ± 33.0 mm³ (p<0.01, ANOVA and post-hoc Dunnett's), 95.5 ± 26.5 mm³ (p<0.01, ANOVA and post-hoc Dunnett's), and 20.8 ± 15.9 mm³ (p<0.01, ANOVA and post-hoc Dunnett's) respectively as compared to vehicle (mean change of tumor volume of 557.7 ± 79.0 mm³) in the second experiment. (See Figure 3)

Compound A was well tolerated at 12.5 and 25 mg/kg as demonstrated by the non-statistically significant mean body weight change for the vehicle treated group (0.1 ± 1.2%) and the Compound A treated group (1.4 ± 1.2% and -1.2 ± 1.2% respectively). However, the group treated with 50 mg/kg of Compound A showed a statistically significant mean change of body weight of 10.6 ± 4.1 % (p<0.05, using a paired t-test) and 8.2 ± 2.6% (p<0.05, using a paired t-test) in the two experiments. (See Figure 2)

### Example 2 (reference) - Effect of Compound A in NCI-N87 gastric cancer xenograft model

Experiments were performed in female HsdNpa:Athymic Nude-nu mice approximately 8-12 weeks of age at treatment start. All animals were housed under Optimized Hygienic conditions in Makrolon type III cages (max. 10 animals per cage) with free access to food and water.

NCI-N87 cells (obtained from the American Type Culture Collection), which are of gastric origin and ErbB2 amplified, were grown in DMEM culture medium containing 4.5g/l glucose supplemented with 10% heat-inactivated FCS, 2mM L-glutamine, and 1 mM sodium pyruvate. The cells were incubated at 37°C in a 5% CO₂ humidified atmosphere. Cells were harvested with trypsin (0.25% w/v)-EDTA (0.53mM), re-suspended in culture medium (with additives) and counted with a Casy® system. Cell culture reagents were purchased from BioConcept (Allschwil, Switzerland).

NCI-N87 tumors were established from fragments of approximately 40mg implanted subcutaneously into the female HsdNpa:Athymic Nude-nu mice with a trocar needle. Treatment started when the tumors reached a size of approximately 110mm³, 25 days post implantation.

Compound A was formulated in NMP/PEG300/Solutol HS15/water (10:30:20:40% vol/vol). The Compound was fully dissolved in NMP. Then, PEG300 and liquified Solutol were added (in sequence with vortexing after addition of each reagent). Water was added immediately prior to administration to the animals.

Tumor volumes were measured with calipers and determined according to the formula: length x diameter² x π/6. Antitumor activity is expressed as T/C % (mean change of tumor volume of treated animals / mean change of tumor volume of control animals) x 100. Regressions (%) were calculated according to the formula ((mean tumor volume at end of treatment-mean tumor volume at start of treatment)/mean tumor volume at start of treatment) x 100. Body weights and tumor volumes were recorded twice a week.

Where applicable, data is presented as mean ± SEM. For all tests, the level of significance was set at p<0.05. For tumor volumes, comparisons between treatment groups and vehicle control group were done using one-way ANOVA followed by Dunnett's test. The level of significance of body weight change within a group between the start and the end of the treatment period was determined using a paired t-test. Comparisons of delta body weights between treatment and vehicle control groups were performed by a one-way ANOVA followed by a post hoc Dunnett's test.

Compound A was orally administered daily to NCI-N87 tumor bearing animals at a dose of 50 mg/kg. Treatments started 18 days post tumor cell implantation and lasted for 20 consecutive days. Vehicle controls consisted of animals receiving a daily oral administration of a mixture of NMP/PEG300/Solutol HS15/water (10:30:20:40 % vol/vol). Compound A produced a statistically significant antitumor effect (p<0.05, ANOVA) with 11% regressions. Compound A produced a mean change of tumor volume of -11.8 ± 17.2 mm³ (p<0.01, ANOVA and post-hoc Dunnett's) as compared to vehicle (mean change of tumor volume of 694.0 ± 93.5 mm³. Compound A was well tolerated as demonstrated by the non-statistically significant mean change of body weight for the group treated with 50 mg/kg of Compound A (3.8 ± 2.1 %) in the experiment. (See Figures 4 and 5.)

### Example 3 - Effect of the combination of Compound A and trastuzumab in BT474 breast cancer xenograft model

Experiments were performed in female HsdNpa:Athymic Nude-nu mice approximately 8-12 weeks of age at treatment start. All animals were housed under Optimized Hygienic conditions in Makrolon type III cages (max. 10 animals per cage) with free access to food and water.

BT-474 cells, which are human breast ductal carcinoma cells and ErbB2 amplified with a PIK3CA mutation K111N, were grown in DMEM culture medium containing 4.5g/l glucose supplemented with 10% heat-inactivated FCS, 2mM L-glutamine, 1 mM sodium pyruvate and incubated at 37°C in a 5% CO₂ humidified atmosphere. Cell culture reagents were purchased from BioConcept (Allschwil, Switzerland).

BT-474 tumors were established in vivo by injection 3x10⁶ cells in 30µl HBSS (Sigma #H8264) containing 1mg/ml Matrigel (BD # 34234) orthotopically into the 3^{rd} mammary gland on the right side of the animals. The efficacy experiments started when the tumors reached an average size of approximately 130 mm³ (day 14 post cell injection). On the day of cell injection, a 0.025 17β Estradiol pellent has been implanted subcutaneously on the left flank into each experimental animal under Forene(R) inhalation narcosis.

Compound A was formulated in NMP/PEG300/Solutol HS15/water (10:30:20:40% vol/vol). The Compound was fully dissolved in NMP. Then, PEG300 and liquified Solutol were added (in sequence with vortexing after addition of each reagent). Water was added immediately prior to administration to the animals. Compound A or vehicle was administered orally at a volume of 10ml/kg. Trastuzumab was reconstituted in PBS and administered intraperitoneally three times per week at a concentration of 3 mg/kg.

Tumor volumes were measured with calipers and determined according to the formula: length x diameter² x π/6. Antitumor activity is expressed as T/C % (mean change of tumor volume of treated animals / mean change of tumor volume of control animals) x 100.

Regressions (%) were calculated according to the formula ((mean tumor volume at end of treatment-mean tumor volume at start of treatment)/mean tumor volume at start of treatment) x 100. Body weights and tumor volumes were recorded twice a week.

Where applicable, data is presented as mean ± SEM. For all tests, the level of significance was set at p<0.05. For tumor volumes, comparisons between treatment groups and vehicle control group were done using one-way ANOVA followed by Dunnett's test. The level of significance of body weight change within a group between the start and the end of the treatment period was determined using a paired t-test. Comparisons of delta body weights between treatment and vehicle control groups were performed by a one-way ANOVA followed by a post hoc Dunnett's test.

In addition, an approximation of drug interactions was made using the method described in Clarke R., Breast Cancer Res. Treat (1997) 46:255-278. This was applied to change in tumor volumes and known to be useful for estimating interactions from limited data. According to the method described by Clarke: For compound A, B or the combination AB (with control group C), antagonism is predicted when the calculation AB/C > A/C x B/C, additive effect when AB/C = A/C x B/C, and synergistic when A x B/C < A/C x B/C.

Compound A was administered orally daily as a single agent to BT-474 tumor bearing animals at a dose of 12.5 mg/kg. Trastuzumab as a single agent was administered as intraperitoneal injections of 3 mg/kg, three times per week. Compound A produced a statistically significant antitumor effect (p<0.05, ANOVA), with a T/C of 21.5%. Trastuzumab also produced a statistically significant antitumor effect (p<0.05, ANOVA), with a T/C of 35.8%. Analysis of the combination interactions with the method described by Clarke R., Breast Cancer Res. Treat (1997) 46:255-278, indicated a synergistic antitumor effect of the combination at 12.5 mg daily and 3 mg/kg three times per week as compared to the single agent. (See Figure 6)

| | C | A (Trastuzumab) | B (Comp. A) | AB (Combo) | A/C | B/C | A/C x B/C | AB/C | Diff. | Result |
|---|---|---|---|---|---|---|---|---|---|---|
| Change Tumor Volume | 584.2 | 209.2 | 125.7 | -2.7 | 0.358 | 0.215 | 0.077 | -0.044 | -0.12 | synergy |

However, this combination was only statistically different from Compound A as single agent (Mann-Whitney Rank Sum Test) and not from trastuzumab as single agent. Treatments were well tolerated since no significant body weight loss was observed.

Compound A was administered orally daily as a single agent to BT-474 tumor bearing animals at a dose of 50 mg/kg. Trastuzumab as a single agent was administered as intraperitoneal injections of 10 mg/kg, three times per week. Compound A produced a statistically significant antitumor effect (p<0.05, Mann-Whitney Rank Sum Test). Trastuzumab also produced a statistically significant antitumor effect (p<0.05, Mann-Whitney Rank Sum Test. ). Analysis of the combination interactions with the method described by Clarke R., Breast Cancer Res. Treat (1997) 46:255-278, indicated a synergistic antitumor effect of the combination at 50 mg daily of Compound A and 10 mg/kg three times per week of trastuzumab as compared to the single agent. (See Figure 7).

A significant body weight loss was observed with Compound A as single agent or in combination with trastuzumab (about 12% and about 10%, respectively).

### Example 4 (reference)- Effect of Compound A alone and in combination with trastuzumab in NCI-N87 gastric cancer xenograft model

Experiments were performed in female CB17 SCID mice (Fox Chase SCID®, CB17/ Icr-*Prkdc*^{scid}, Charles River) approximately 7-8 weeks of age and having a body weight (BW) range of 14.7-21.2 g at treatment start. All animals were fed ad libitum water (reverse osmosis, 1 ppm Cl) and NIH 31 Modified and Irradiated Lab Diet® consisting of 18.0% crude protein, 5.0% crude fat, and 5.0% crude fiber. The mice were housed on irradiated Enrich-o'cobs™ Laboratory Animal Bedding in static microisolators on a 12-hour light cycle at 21-22°C and 40-60% humidity.

NCI-N87 cells (obtained from the American Type Culture Collection), which are of gastric origin and ErbB2 amplified, were maintained as exponentially growing cultures in RPMI-1640 medium supplemented with 10% fetal bovine serum, 2 mM glutamine, 100 units/ mL penicillin G sodium, and 100 µg/mL streptomycin sulfate. The tumor cells were cultured in tissue culture flasks in a humid incubator at 37°C, in an atmostphere of 5% CO₂ and 95% air. Cells were harvested with 1X trypsin and suspended at a concentration of 5 x 10⁷ cells/ mL in cold phosphate-buffered saline with 50% Matrigel. Each mouse was injected subcutaneously in the right flank with 1 x 10⁷ cells (0.2 mL cell suspension). Tumor volumes were measured with calipers and determined according to the formula: width² x length /2, and tumor weight estimated with the assumption that 1 mg is equivalent to 1 mm³ of tumor volume. Nine days after tumor implantation (on Day 1 of the study), mice with individual tumor volumes of 126- 320 mm³ were sorted into 12 groups of eight mice with a group mean tumor volume of 231-239 mm³.

Compound A was formulated in NMP/PEG300/Solutol HS15/deionized water (10:30:20:40% vol/vol). The Compound was fully dissolved in NMP. Then, PEG300 and liquified Solutol were added (in sequence with vortexing after addition of each reagent). Water was added prior to administration to the animals. Fresh dosing solutions were prepared weekly and stored at 4°C, protected from light.

Trastuzumab (Herceptin®, Genentech, 21 mg/mL) was freshly diluted with saline (Vehicle 2) on each day of dosing.

Treatments started 9 days post tumor cell implantation and lasted for 30 consecutive days (or until tumor volume = 2000 mm³). Compound A was orally administered once daily to NCI-N87 tumor bearing animals, and trastuzumab was administered by intraperitoneal injection (i.p.) twice weekly for four weeks. Controls (Group 1) consisted of animals receiving a mixture of NMP/PEG300/Solutol HS15/water (10:30:20:40 % vol/vol) (Vehicle 1) per oral (p.o.) once daily and saline (Vehicle 2) by intraperitoneal injection (i.p.) twice weekly for four weeks. For combination therapies, trastuzumab was dosed within 30 minutes after Compound A. Dosing volume (10 mL/kg, was scaled to the weight of each animal as determined on day of dosing, except on weekends when the previous BW was carried forward. Groups 2-4 received monotherapies with 12.5, 25 and 50 mg/kg Compound A, respectively. Groups 5 and 6 received monotherapeis with 3 and 10 mg/kg trastuzumab, respectively. Groups 7-9 received 12.5, 25 and 50 mg/kg Compound A, respectively, each in combination with 3 mg/kg trastuzumab. Groups 10-12 received 12.5, 25 and 50 mg/kg Compound A, respectively, each in combination with 10 mg/kg trastuzumab. Groups 4, 9 and 12 were stopped early. Body weights were recorded on Days 1-5, on each treatment day (except weekends), and twice weekly thereafter until end of the study.

Antitumor activity is expressed as T/C % (mean change of tumor volume of treated animals / mean change of tumor volume of control animals) x 100. A treatment that achieves a T/C value of 40% or less would be classified as potentially therapeutically active. Treatment efficacy may also be determined from the number of regression responses: (a) a partial regression (PR) indicates that the tumor was 50% or less of its starting Day 1 volume for three consecutive measurements in the study and equal to or greater than 13.5 mm3 for one or more of these three measurements, and (b) a complete regression (CR) indicates that the tumor volume was less than 13.5 mm³ for three consecutive measurements during the study.

Where applicable, data is presented as mean ± SEM. Statisticial and graphical analyses was performed with Prism 3.03 (GraphPad) for Windows. For all tests, the level of significance of differences was done using ANOVA, with Bartlett's test; and a post-hoc Dunnett's multiple comparison test compared the mean change for each drub-treated group to the mean for Group 1. When Bartlett's test for equal variance showed significant differences (P = 0.0401), groups were compared with the nonparametric Kruskal-Wallis test, which showed significant differences among the median volume changes (P < 0.0001). The significance of differences between the median volume changes for drug-treated grups and Group 1 was analyzed post hoc with Dunn's multiple comparison test. The two-tailed statistical analyses were conducted at P = 0.05. Prism summarizes test results as not significant (ns) at P > 0.05, significant (symbolized by "*") at 0.01 < P ≤ 0.05, very significant (symbolized by "**") at 0.001 < P ≤ 0.01, and extremely significant (symbolized by "***") at P ≤ 0.001.

The following results were obtained with the statistical significance calculated with Kruskal-Wallis with Post-hoc Dunn's multiple comparison test:

| ***Group*** | ***No***. ***ani mal s*** | ***T***/***C or T*/*T₀*** | ***Statistical Significance*** | | | | | ***Regress ions*** | ***Mean BW Nadir (lowest group mean BW*)** | ***Deaths (TR*** = ***treatment-related***, ***NTR* = *non-treatment related)*** |
|---|---|---|---|---|---|---|---|---|---|---|
| | | | ***vs. G1*** | ***vs. G2*** | ***vs. G3*** | ***vs. G5*** | ***vs*. *G6*** | | | |
| 1 | 8 | -- | -- | -- | -- | -- | -- | PR: 0, CR: 0 | -- | 0 |
| 2 | 8 | 46% | ns | -- | -- | -- | -- | PR: 0, CR: 0 | -7% (Day 22) | 0 |
| 3 | 6 | 12% | * | -- | -- | -- | -- | PR: 0, CR: 0 | -12.2% (Day 26) | TR: 1 |
| | | | | | | | | | | NTR: 1 |
| 4 | 4 | 81% | ne | -- | -- | -- | -- | PR: 0, CR: 0 | -13.1% (Day 12) | TR: 4 NTR: 0 |
| 5 | 8 | 50% | ns | -- | -- | -- | -- | PR: 0, CR: 0 | -- | 0 |
| 6 | 8 | 12% | ** | -- | -- | -- | -- | PR: 0, CR: 0 | -- | 0 |
| 7 | 8 | 15% | * | ** | -- | ** | -- | PR: 0, CR: 0 | -8% (Day 22) | 0 |
| 8 | 4 | 19% | ne | -- | ne | ne | -- | PR: 0, CR: 0 | -6.1% (Day 22) | TR: 4 |
| | | | | | | | | | | NTR: 0 |
| 9 | 5 | 22% | ne | -- | ne | -- | -- | PR: 3, | -11.2% (Day | TR: 3 |
| | | | | | | | | CR: 0 | 19) | NTR: 0 |
| 10 | 8 | -61% | *** | *** | -- | -- | * | PR: 7, CR: 0 | -1.7% (Day 26) | 0 |
| 11 | 8 | -69% | *** | -- | * | -- | ** | PR: 6, CR: 0 | -12.5% (Day 26) | 0 |
| 12 | 5 | 2% | ne | -- | -- | -- | -- | PR: 2, CR: 0 | -12.9% (day 19) | TR: 3 |
| | | | | | | | | | | NTR: 0 |

For Groups 7-9 treated with a combination of Compound A and 3 mg/kg trastuzumab, Group 7 produced significantly stronger inhibition that the corresponding monotherapies I Groups 2 and 5 (P < 0.01). Groups 8 and 9 were excluded from statistical evaluations due to the small final group size.

For Groups 10-12 treated with a combination of Compound A and 10 mg/kg trastuzuab, Group 10 produced significantly stronger activity than the Compound A monotherapy in Group 2 and trastuzumab monotherapy in Group 6. Group 11 produced significant activity (P < 0.001), and 6 partial regressions. The Group 6 combination was significantly superior to Compound A monotherapy in Group 3 and trastuzumab monotherapy in Group 6. Group 12 was excluded from statistical evaluations due to the small final group size.

### Example 5 - Effect of Compound A alone and in combination with trastuzumab in HBCx-5 breast cancer xenograft model

Experiments were performed in female HSD: athymic nude-Foxn1nu mice approximately 8-10 weeks of age and weighing approximately 19-25 grams. The mice were allowed to acclimate with access to food and water ad libitum for 6 days prior to the study. When body weight loss reaches 15% compared to the first day of treatment, DietGel® was provided to the animals.

The mice were divided into five groups each comprising ten mice. Treatments were initiated 34 days post-implantation of the HBCx-5 xenograft tumor. HBCx-5 is a xenograft derived from primary breast carcinoma. It is a ductal adenocarcinoma with wild-type TP53, high RB expression and HER2 overexpression. For xenograft implantation, the mice were anesthetized with 100 mg/kg ketamine hydrochloride and 10 mg/kg xylazine. Using asceptic technique, the mice were grafted in interscapular with 3 mm on 3 mm tumor fragment. Animals were randomized according to tumor volume such that the mean tumor volume and range were statistically similar between treatment groups. Tumors were measured with calipers three times per week at the start of dosing. Tumors were calculated using the formula: = width² x length /2. Tumor size and body weight were measured three times per week during the treatment period. The planned endpoints for the experiment were a treatment phase of six weeks and no follow-up phase.

Compound A was suspended at mg/ml in 0.5% methylcellulose (Sigma Aldrich, St. Louis, MO). It was homogenized by stirring vortexing and sonication. The suspension was stored at 4°C for 7 days protected from light. Trastuzumab (Herceptin®, Roche) solution at 1 mg/ml was prepared on each administration day. Stock solution at 21 mg/ml was diluted just before administration at 1 mg/ml with sterile water for injection. Capecitabine (Xeloda®, Roche) at 540 mg/ml was kept at 4°C protected from light. Capecitabine pellets were crushed and suspended in NaCl 0.9%.

Compound A was administered daily by oral gavage (p.o.) at 50 mg/kg for 42 days, alone or in combination with trastuzumab. Trastuzumab was administered at 10 mg/kg, i.p., once a week for 6 weeks. Dose was body weight adjusted at each injection.

Capecitabine was administered by oral gavage (p.o.) at 540 mg/ml, five times per week for two weeks (i.e., five consecutive dosing days/ week). After a one week rest, a second treatment cycle was performed.

Antitumor activity is expressed as T/C % (mean change of tumor volume of treated animals / mean change of tumor volume of control animals) x 100. Tumor volume and relative body weight were used for statistical analysis. Group comparisons were performed using a Mann Whitney non parametric test between treated group and control group.

In this study, Compound A administered as 50 mg/kg single agent demonstrated a significant antitumor activity on HBCx-5 breast xenograft model (T/C = 17.57%, p < 0.001). Trastuzumab administered at 10 mg/kg single agent did not demonstrate any significant antitumor activity (T/C - 81.44%). Compound A at 50 mg/kg in combination with trastuzumab at 10 mg/kg demonstrated a significant antitumor activity (T/C - 19.44%, p<0.001). However, the antitumor activity of the combined dosing was not significantly different to the Compound A single agent. No partial or complete regressions were observed.

Compound A, used alone or in combination with trastuzumab, was well tolerated. A maximal body weight loss of 9.5% was reached 10 days after first dosing and stabilized until end of study. This weight loss was significant but acceptable. No significant weight loss was observed for trastuzumab alone. When compound A was administered in combination with trastuzumab, a significant but tolerable weight loss of 7.9% was observed.

Capecitabine administered as 40 mg/kg single agent was very active in HBCx-5 breast xenograft model (T/C = 9.03%, p < 0.001). Capecitabine induced a significant and transient body weight loss of 9.1%.

### Example 6 (reference)- Effect of Compound A alone and in combination with lapatinib in NCI-N87 gastric cancer xenograft model

Using the study protocol described in Example 4, the effect of Compound A was studied as monotherapy and in combination with lapatinib in NCI-N87 gastric cancer CB17 SCID mouse xenograft model. The female CB17 SCID mice (Fox Chase SCID® , CB17/ Icr-*Prkdc*^{scid}, Charles River) were 10 weeks old and had a body weight (BW) range of 15.8-21.9 g on day 1 of the study. The NCI-N87 cells were harvested with 5X trypsin. Eight days after tumor implantation (on Day 1 of the study), mice with an individual tumor volume of 88-196 mm³ were sorted into 10 groups of ten mice with a group mean tumor volume of 143-149 mm³.

Replacing the trastuzumab used in Example 4, lapatinib (Tykerb®, GlaxoSmithKline, 250 mg tablet) was suspended in 0.5% hydroxypropyl methylcellulose: 0.1% Tween® 80: 99.4% deionized water (Vehicle 2) for dosing. A fresh lapatinib suspension was prepared once weekly, stored at 4°C, and protected from light.

Treatments started eight days post tumor cell implantation and lasted for 59 consecutive days or until tumor volume = 1000 mm³. Compound A was orally administered once daily to NCI-N87 tumor bearing animals, and lapatinib was orally administered either twice daily (b.i.d.) for 21 days or once daily for 21 days. For the b.i.d. dosing schedule, the first dose was given p.m. on Day 1 and last dose a.m. on Day 22. For combiantin therapies, lapatinib was dosed within 30 minutes after Compound A. Dosing volume (10 mL/kg, was scaled to the weight of each animal as determined on day of dosing, except on weekends when the previous BW was carried forward. Controls (Group 1) consisted of animals receiving a mixture of NMP/PEG300/Solutol HS15/water (10:30:20:40 % vol/vol) (Vehicle 1) per oral (p.o.) once daily and 0.5% hydroxypropyl methylcellulose: 0.1 % Tween® 80: 99.4% deionized water (Vehicle 2) per oral once daily. Groups 2 and 3 received monotherapies with 12.5 and 25 mg/kg Compound A, respectively. Group 4 received lapatinib monotherapy at 50 mg/kg b.i.d. Groups 5 and 6 received monotherapiess with 100 and 150 mg/kg lapatinib, respectively, once daily. Groups 7 and 8 received 12.5 and 25 mg/kg Compound A, respectively, once daily, each in combination with lapatinib once daily. Group 9 received 12.5 mg/kg Compound A once daily in combination with 50 mg/kg lapatinib b.i.d., wherein Compound A was dosed together with the p.m. dose of lapatinib. Group 10 received 12.5 mg/kg Compound A once daily in combination with 150 mg/kg lapatinib once daily. Treatment for Groups 7, 8, 9 and 10 was terminated early. Body weights were recorded on Days 1-5, on each treatment day (except weekends), and twice weekly thereafter until end of the study.

Where applicable, data is presented as mean ± SEM. Each animal was euthanized when its neoplasm reached the endpoint volume (1000 mm³), or on the last day of the study (Day 59). Time to endpoint (TTE) is calculated by the equation: TTE = (log₁₀(endpoint volume) - b) / m, where TTE is expressed in days, endpoint volume is in mm3, b is the intercept and m is the slope of the line obtained by linear regression of a log-transformed tumor growth data set. Treatment efficacy was determined from Tumor Growth Delay (TGD), which was defined as the increase in the median time to endpoint (TTE) for a treatment group compared to the control group (TGD = T-C), expressed in days, or as a percentage of the median TTE of the control group (%TGD = ((T-C)/C) x 100), wherein T = median TTE for a treatment group and C = median TTE for the designated control group.

Statistical and graphical analyses was performed with Prism 3.03 (GraphPad) for Windows. For all tests, the level of significance of differences was done using ANOVA, with Bartlett's test; and a post-hoc Dunnett's multiple comparison test compared the mean change for each drub-treated group to the mean for Group 1. Survival was analyzed by the Kaplan-Meier method. The logrank test was employed to analyze the significance of the difference between the overall survival experiences of two groups, based on time to endpoint (TTE) values. The two-tailed statistical analyses were conducted at P = 0.05. Prism summarizes test results as not significant (ns) at P > 0.05, significant (symbolized by "*") at 0.01 < P ≤ 0.05, very significant (symbolized by "**") at 0.001 < P ≤ 0.01, and extremely significant (symbolized by "***") at P ≤ 0.001.

The following results were obtained with the statistical significance calculated with the Logrank test:

| ***Gro up*** | ***No. ani mal s*** | **Median TTE** | **T-C** | **% *TGD*** | ***Statis tical Signif*** . ***vs. G1*** | ***Mean Tumor Volume (no. animals** , **n), Day 59*** | ***Regre ssions*** | ***Mean BW Nadir (lowest group mean BW)*** | ***Deaths (TR* = *treatment -related, NTR* = *non-treatment related)*** |
|---|---|---|---|---|---|---|---|---|---|
| 1 | 10 | 26.2 | -- | -- | -- | -- | PR: 0, CR: 0 | -0.4% | 0 |
| | | | | | | | | Day 14 | |
| 2 | 9 | 30.4 | 4.2 | 16 | ns | -- | PR: 0, CR: 0 | -- | TR: 1 |
| | | | | | | | | | NTR: 1 |
| 3 | 9 | 43.5 | 17.3 | 16 | * | -- | PR: 0, CR: 0 | -8.4% | TR: 1 |
| | | | | | | | | Day 14 | NTR: 1 |
| 4 | 10 | 47.7 | 21.5 | 66 | *** | 608 (1) | PR: 0, CR: 0 | -8.1% | 0 |
| | | | | | | | | Day 21 | |
| 5 | 10 | 42.6 | 16.4 | 82 | ** | 750 (1) | PR: 0, CR: 0 | -1.4% | 0 |
| | | | | | | | | Day 17 | |
| 6 | 9 | 50.6 | 24.4 | 63 | *** | -- | PR: 1, CR: 0 | -10.4% | TR: 0 |
| | | | | | | | | Day 14 | NTR: 1 |
| 7 | 10 | 36.5 | 10.3 | 93 | ne | -- | PR: 0, CR: 0 | -6.1% | TR: 3 |
| | | | | | | | | Day 14 | NTR: 0 |
| 8 | 10 | 33.6 | 7.4 | 39 | ne | -- | PR: 1, CR: 0 | -7.7% | TR: 4 |
| | | | | | | | | Day 7 | NTR: 0 |
| 9 | 10 | 39.1 | 12.9 | 28 | ne | 650 (1) | PR: 1, CR: 0 | -11.3% | TR: 4 |
| | | | | | | | | Day 10 | NTR: 0 |
| 10 | 9 | 8.0 | -18.2 | 49 | ne | -- | PR: 0, CR: 0 | -7% | TR: 6 |
| | | | | | | | | Day 7 | NTR: 1 |

## Claims

1. Use of a compound (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide), or a pharmaceutically acceptable salt thereof, together with an EGFR inhibitor for the manufacture of a pharmaceutical preparation for the treatment of an EGFR dependent disease, wherein the disease to be treated is breast cancer.

2. The use according to claim 1, wherein said disease is resistant to the treatment with an EGFR inhibitor.

3. The use according to claim 2, wherein resistance to the treatment with an EGFR inhibitor has been acquired during treatment with said EGFR inhibitor.

4. The use according to claim 2 or 3, wherein the EGFR inhibitor is selected from the group consisting of gefitinib, erlotinib, lapatinib, cetuximab, nimotuzumab, panitumumab, trastuzumab and TDM1.

5. The use according to claim 1, wherein the EGFR inhibitor is selected from the group consisting of gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, TDM1, Zemab®, the Her2 vaccine PX 1041, and the HSP90 inhibitors CNF1010, CNF2024, tanespimycin, alvespimycin, IPI504, SNX5422 and NVP-AUY922.

6. The use according to claim 1 wherein the EGFR inhibitor is trastuzumab.

7. The compound (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide), or a salt thereof, in combination with an EGFR inhibitor for use in the treatment of an EGFR dependent disease, wherein the disease to be treated is breast cancer.

8. The compound for use according to claim 7, wherein the disease is resistant to the treatment with an EGFR inhibitor.

9. The compound (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide), or a salt thereof, in combination with trastuzumab for use in the treatment of an EGFR dependent disease, wherein the disease to be treated is breast cancer.

10. Combination of (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) and an EGFR modulator selected from the group consisting of gefitinib, erlotinib, lapatinib, NVP-AEE778, ARRY334543, BIRW2992, BMS690514, pelitinib, vandetanib, AV412, anti-EGFR monoclonal antibody 806, anti-EGFR monoclonal antibody-Y90/Re-188, cetuximab, panitumumab, matuzumab, nimotuzumab, zalutumumab, pertuzumab, MDX-214, CDX110, IMC11F8, pertuzumab, trastuzumab, TDM1, Zemab®, the Her2 vaccine PX 1041, and the HSP90 inhibitors CNF1010, CNF2024, tanespimycin, alvespimycin, IPI504, SNX5422 and NVP-AUY922, wherein the active ingredients are present in each case in free form or in the form of a pharmaceutically acceptable salt, and optionally at least one pharmaceutically acceptable carrier, for simultaneous, separate or sequential use in the treatment of breast cancer.

11. The combination for use according to claim 10, wherein the EGFR modulator is trastuzumab.

12. A compound (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide) for use in a method of treating an EGFR dependent disease or a disease that has acquired resistance during treatment with an EGFR inhibitor, wherein the disease to be treated is breast cancer.

13. A compound for use according to claim 12, wherein the compound is administered together with an EGFR inhibitor according to claim 5.

14. A pharmaceutical preparation for use in the treatment of an EGFR dependent disease or a disease that has acquired resistance during treatment with an EGFR inhibitor comprising a compound (S)-Pyrrolidine-1,2-dicarboxylic acid 2-amide 1-({4-methyl-5-[2-(2,2,2-trifluoro-1,1-dimethyl-ethyl)-pyridin-4-yl]-thiazol-2-yl}-amide), according to claim 1 or a pharmaceutically acceptable salt thereof and at least one pharmaceutically acceptable carrier, wherein the disease to be treated is breast cancer.

15. The pharmaceutical preparation for use according to claim 14, comprising an EGFR inhibitor according to claim 5.

## Patentansprüche

1. Verwendung einer Verbindung (S)-Pyrrolidin-1,2-dicarbonsäure-2-amid-1-({4-methyl-5-[2-(2,2,2-trifluor-1,1-dimethyl-ethyl)-pyridin-4-yl]-thiazol-2-yl}-amid), oder eines pharmazeutisch akzeptablen Salzes davon, zusammen mit einem EGFR-Inhibitor zur Herstellung einer pharmazeutischen Zubereitung zur Behandlung einer EGFR-abhängigen Erkrankung, wobei die zu behandelnde Erkrankung Brustkrebs ist.

2. Verwendung nach Anspruch 1, wobei die Erkrankung gegenüber der Behandlung mit einem EGFR-Inhibitor resistent ist.

3. Verwendung nach Anspruch 2, wobei die Resistenz gegenüber der Behandlung mit einem EGFR-Inhibitor während der Behandlung mit dem EGFR-Inhibitor erworben wurde.

4. Verwendung nach Anspruch 2 oder 3, wobei der EGFR-Inhibitor ausgewählt ist aus der Gruppe, bestehend aus Gefitinib, Erlotinib, Lapatinib, Cetuximab, Nimotuzumab, Panitumumab, Trastuzumab und TDM1.

5. Verwendung nach Anspruch 1, wobei der EGFR-Inhibitor ausgewählt ist aus der Gruppe, bestehend aus Gefitinib, Erlotinib, Lapatinib, NVP-AEE778, ARRY334543, BIRW2992, BMS690514, Pelitinib, Vandetanib, AV412, monoklonalem anti-EGFR-Antikörper 806, monoklonalem anti-EGFR-Antikörper-Y90/Re-188, Cetuximab, Panitumumab, Matuzumab, Nimotuzumab, Zalutumumab, Pertuzumab, MDX-214, CDX110, IMC11F8, Pertuzumab, Trastuzumab, TDM1, Zemab®, dem Her2-Impfstoff PX 1041 und den HSP90-Inhibitoren CNF1010, CNF2024, Tanespimycin, Alvespimycin, IPI504, SNX5422 und NVP-AUY922.

6. Verwendung nach Anspruch 1, wobei es sich bei dem EGFR-Inhibitor um Trastuzumab handelt.

7. Verbindung (S)-Pyrrolidin-1,2-dicarbonsäure-2-amid-1-({4-methyl-5-[2-(2,2,2-trifluor-1,1-dimethylethyl)pyridin-4-yl]thiazol-2-yl}amid) oder ein Salz davon in Kombination mit einem EGFR-Inhibitor zur Verwendung in der Behandlung einer EGFR-abhängigen Erkrankung, wobei die zu behandelnde Erkrankung Brustkrebs ist.

8. Verbindung zur Verwendung nach Anspruch 7, wobei die Erkrankung gegenüber der Behandlung mit einem EGFR-Inhibitor resistent ist.

9. Verbindung (S)-Pyrrolidin-1,2-dicarbonsäure-2-amid-1-({4-methyl-5-[2-(2,2,2-trifluor-1,1-dimethylethyl)pyridin-4-yl]thiazol-2-yl}amid) oder ein Salz davon in Kombination mit Trastuzumab zur Verwendung in der Behandlung einer EGFR-abhängigen Erkrankung, wobei die zu behandelnde Erkankung Brustkrebs ist.

10. Kombination von (S)-Pyrrolidin-1,2-dicarbonsäure-2-amid-1-({4-methyl-5-[2-(2,2,2-trifluor-1,1-dimethyl-ethyl)-pyridin-4-yl]-thiazol-2-yl}-amid) und einem EGFR-Modulator, ausgewählt aus der Gruppe, bestehend aus Gefitinib, Erlotinib, Lapatinib, NVP-AEE778, ARRY334543, BIRW2992, BMS690514, Pelitinib, Vandetanib, AV412, monoklonalem anti-EGFR-Antikörper 806, monoklonalem anti-EGFR-Antikörper-Y90/Re-188, Cetuximab, Panitumumab, Matuzumab, Nimotuzumab, Zalutumumab, Pertuzumab, MDX-214, CDX110, IMC11F8, Pertuzumab, Trastuzumab, TDM1, Zemab®, dem Her2-Impfstoff PX 1041 und den HSP90-Inhibitoren CNF1010, CNF2024, Tanespimycin, Alvespimycin, IPI504, SNX5422 und NVP-AUY922, wobei die Wirkstoffe in jedem Fall in freier Form oder in der Form eines pharmazeutisch akzeptablen Salzes vorliegen, und gegebenenfalls mindestens einem pharmazeutisch akzeptablen Träger zur simultanen, separaten oder nacheinander erfolgenden Verwendung bei der Behandlung von Brustkrebs.

11. Kombination zur Verwendung nach Anspruch 10, wobei der EGFR-Modulator Trastuzumab ist.

12. Verbindung (S)-Pyrrolidin-1,2-dicarbonsäure-2-amid-1-({4-methyl-5-[2-(2,2,2-trifluor-1,1-dimethyl-ethyl)-pyridin-4-yl]-thiazol-2-yl}-amid) zur Verwendung bei einem Verfahren zur Behandlung einer EGFR-abhängigen Erkrankung oder einer Erkrankung, die während der Behandlung mit einem EGFR-Inhibitor Resistenz erworben hat, wobei die zu behandelnde Erkrankung Brustkrebs ist.

13. Verbindung zur Verwendung nach Anspruch 12, wobei die Verbindung zusammen mit einem EGFR-Inhibitor nach Anspruch 5 verabreicht wird.

14. Pharmazeutische Zubereitung zur Verwendung bei der Behandlung einer EGFR-abhängigen Erkrankung oder einer Erkrankung, die während der Behandlung mit einem EGFR-Inhibitor Resistenz erworben hat, umfassend eine Verbindung (S)-Pyrrolidin-1,2-dicarbonsäure-2-amid-1-({4-methyl-5-[2-(2,2,2-trifluor-1,1-dimethyl-ethyl)-pyridin-4-yl]-thiazol-2-yl}-amid) nach Anspruch 1 oder ein pharmazeutisch akzeptables Salz davon und mindestens einen pharmazeutisch akzeptablen Träger, wobei die zu behandelnde Erkrankung Brustkrebs ist.

15. Pharmazeutische Zubereitung zur Verwendung nach Anspruch 14, umfassend einen EGFR-Inhibitor nach Anspruch 5.

## Revendications

1. Utilisation d'un composé qui est le 2-amide et 1-(14-méthyl-5-[2-(2,2,2-trifluoro-1,1-diméthyl-éthyl)-pyridin-4-yl]-thiazol-2-yl}-amide) d'acide (S)-pyrrolidine-1,2-dicarboxylique, ou un sel pharmaceutiquement acceptable de celui-ci, avec un inhibiteur d'EGFR dans la fabrication d'une préparation pharmaceutique destinée au traitement d'une maladie EGFR-dépendante, où la maladie à traiter est un cancer du sein.

2. Utilisation selon la revendication 1, où ladite maladie est résistante au traitement par un inhibiteur d'EGFR.

3. Utilisation selon la revendication 2, où la résistance au traitement par un inhibiteur d'EGFR a été acquise pendant le traitement par ledit inhibiteur d'EGFR.

4. Utilisation selon la revendication 2 ou 3, où l'inhibiteur d'EGFR est choisi dans le groupe constitué par les suivants : géfitinib, erlotinib, lapatinib, cétuximab, nimotuzumab, panitumumab, trastuzumab et TDM1.

5. Utilisation selon la revendication 1, où l'inhibiteur de EGFR est choisi dans le groupe constitué par les suivants : géfitinib, erlotinib, lapatinib, NVP-AEE778, ARRY334543, BIRW2992, BMS690514, pélitinib, vandétanib, AV412, anticorps monoclonal anti-EGFR 806, anticorps monoclonal anti-EGFR Y90/Re-188, cétuximab, panitumumab, matuzumab, nimotuzumab, zalutumumab, pertuzumab, MDX-214, CDX110, IMC11F8, pertuzumab, trastuzumab, TDM1, Zemab®, le vaccin Her2 PX 1041, et les inhibiteurs de HSP90 CNF1010, CNF2024, tanéspimycine, alvéspimycine, IPI504, SNX5422 et NVP-AUY922.

6. Utilisation selon la revendication 1, dans laquelle l'inhibiteur d'EGFR est le trastuzumab.

7. Composé 2-amide et 1-({4-méthyl-5-[2-(2,2,2-trifluoro-1,1-diméthyl-éthyl)pyridin-4-yl]-thiazol-2-yl}-amide) d'acide (S)-pyrrolidine-1,2-dicarboxylique, ou un sel de celui-ci, en combinaison avec un inhibiteur d'EGFR pour utilisation dans le traitement d'une maladie EGFR-dépendante, où la maladie à traiter est un cancer du sein.

8. Composé pour utilisation selon la revendication 7, où la maladie est résistante au traitement avec un inhibiteur d'EGFR.

9. Composé 2-amide et 1-({4-méthyl-5-[2-(2,2,2-trifluoro-1,1-diméthyl-éthyl)pyridin-4-yl]-thiazol-2-yl}-amide) d'acide (S)-pyrrolidine-1,2-dicarboxylique, ou un sel de celui-ci, en combinaison avec le trastuzumab pour utilisation dans le traitement d'une maladie EGFR-dépendante, où la maladie à traiter est un cancer du sein.

10. Combinaison de 2-amide et 1-({4-méthyl-5-[2-(2,2,2-trifluoro-1,1-diméthyl-éthyl)-pyridin-4-yl]-thiazol-2-yl}-amide) d'acide (S)-pyrrolidine-1,2-dicarboxylique et d'un modulateur d'EGFR choisi dans le groupe constitué par les suivants : géfitinib, erlotinib, lapatinib, NVP-AEE778, ARRY334543, BIRW2992, BMS690514, pélitinib, vandétanib, AV412, anticorps monoclonal anti-EGFR 806, anticorps monoclonal anti-EGFR Y90/Re-188, cétuximab, panitumumab, matuzumab, nimotuzumab, zalutumumab, pertuzumab, MDX-214, CDX110, IMC11F8, pertuzumab, trastuzumab, TDM1, Zemab®, le vaccin Her2 PX 1041, et les inhibiteurs de HSP90 CNF1010, CNF2024, tanéspimycine, alvespimycine, IPI504, SNX5422 et NVP-AUY922, où les principes actifs sont présents dans chacun sous forme libre ou sous la forme d'un sel pharmaceutiquement acceptable, et éventuellement au moins un vecteur pharmaceutiquement acceptable, pour utilisation simultanée, séparée ou séquentielle dans le traitement du cancer du sein.

11. Combinaison pour utilisation selon la revendication 10, où le modulateur d'EGFR est le trastuzumab.

12. Composé qui est le 2-amide et 1-({4-méthyl-5-[2-(2,2,2-trifluoro-1,1-diméthyl-éthyl)-pyridin-4-yl]-thiazol-2-yl}-amide) d'acide (S)-pyrrolidine-1,2-dicarboxylique pour utilisation dans une méthode de traitement d'une maladie EGFR-dépendante ou d'une maladie ayant acquis une résistance pendant le traitement avec un inhibiteur d'EGFR, où la maladie à traiter est un cancer du sein.

13. Composé pour utilisation selon la revendication 12, où le composé est administré avec un inhibiteur d'EGFR selon la revendication 5.

14. Préparation pharmaceutique pour utilisation dans le traitement d'une maladie EGFR-dépendante ou d'une maladie ayant acquis une résistance pendant le traitement avec un inhibiteur d'EGFR comprenant un composé qui est le 2-amide et 1-({4-méthyl-5-[2-(2,2,2-trifluoro-1,1-diméthyl-éthyl)-pyridin-4-yl]-thiazol-2-yl}-amide) d'acide (S)-pyrrolidine-1,2-dicarboxylique, selon la revendication 1 ou un sel pharmaceutiquement acceptable de celui-ci et au moins un vecteur pharmaceutiquement acceptable, où la maladie à traiter est un cancer du sein.

15. Préparation pharmaceutique pour utilisation selon la revendication 14, comprenant un inhibiteur d'EGFR selon la revendication 5.
